# EUROPEAN PATENT SPECIFICATION

(11) **EP 2 812 444 B1**
(45) Date of publication and mention of the grant of the patent: **28.10.2015**
(21) Application number: 13705233.8
(22) Date of filing: 06.02.2013
(51) Int. Cl.: C12Q 1/00

(54) **ELECTROCHEMICAL-BASED ANALYTICAL TEST STRIP WITH FILL-SPEED CONFIGURED REAGENT LAYER**
ANALYTISCHER TESTSTREIFEN AUF ELEKTROCHEMISCHER BASIS MIT FÜLLGESCHWINDIGKEITSKONFIGURIERTER REAGENZSCHICHT
BANDELETTE RÉACTIVE À BASE ÉLECTROCHIMIQUE AVEC COUCHE DE RÉACTIF CONFIGURÉE AVEC VITESSE DE REMPLISSAGE

(30) Priority: 07.02.2012 US 201213367648
(43) Date of publication of application: 17.12.2014
(73) Proprietor: Lifescan Scotland Limited, Inverness-Shire IV2 3ED (GB)
(72) Inventor: WHITEHEAD, Neil, Inverness IV2 3ED (GB); PHILLIPS, Stuart, Inverness IV2 3ED (GB); MORRIS, David, Inverness IV2 3ED (GB); MCILRATH, Joanne, Inverness IV2 3ED (GB); MACLEOD, Robert, Inverness IV2 3ED (GB); WHYTE, Lynsey, Inverness IV2 3ED (GB); CAMPBELL, Karn, Inverness IV2 3ED (GB); DARLING, Ramsay, Inverness IV2 3ED (GB); MCLAREN, James, Inverness IV2 3ED (GB); BAIN, Russell, Inverness IV2 3ED (GB)
(74) Representative: Brunner, John Michael Owen
(86) International application number: PCT/GB2013/050275
(87) International publication number: WO 2013/117924

(56) References cited:
- EP-A2- 2 182 355
- WO-A2-2004/093784
- GB-A- 2 391 945

## Description

### BACKGROUND OF THE INVENTION

### Field of the Invention

The present invention relates, in general, to medical devices and, in particular, to analytical test strips and related methods.

### Description of Related Art

The determination (e.g., detection and/or concentration measurement) of an analyte in a fluid sample is of particular interest in the medical field. For example, it can be desirable to determine glucose, ketone bodies, cholesterol, lipoproteins, triglycerides, acetaminophen and/or HbA1 c concentrations in a sample of a bodily fluid such as urine, blood, plasma or interstitial fluid. Such determinations can be achieved using analytical test strips, based on, for example, visual, photometric or electrochemical techniques. Conventional electrochemical-based analytical test strips are described in, for example, U.S. Patent Nos. 5,708,247, and 6,284,125.

EP2182355 A2 discloses an electrochemical-based analytical test strip, which includes an electrically-insulating substrate, a patterned conductive layer disposed over the electrically-insulating substrate, a patterned insulating layer disposed over the patterned conductive layer, an enzymatic reagent layer disposed over the patterned conductive layer, a patterned adhesive layer disposed above the enzymatic reagent layer and a top layer disposed over the enzymatic reagent layer.

### BRIEF DESCRIPTION OF THE DRAWINGS

The accompanying drawings, which are incorporated herein and constitute part of this specification, illustrate presently preferred embodiments of the invention, and, together with the general description given above and the detailed description given below, serve to explain features of the invention, in which:
FIG. 1 is a simplified exploded view of an electrochemical-based analytical test strip according to an embodiment of the present invention;
FIG. 2 is a simplified semi-exploded view of the electrochemical-based analytical test strip of FIG. 1;
FIG. 3 is a simplified bottom outline view of a distal end portion of an electrically insulating substrate layer, patterned conductor layer, patterned insulating layer, reagent layer, patterned spacer layer, and hydrophilic layer of the electrochemical-based analytical test strip of FIG. 1;
FIG. 4 is a simplified top outline view of the pattered spacer layer, and hydrophilic layer of the electrochemical-based analytical test strip of FIG. 1;
FIGs. 5A-5C are simplified top views of the patterned spacer layer, hydrophilic layer and top layer of the electrochemical-based analytical test strip of FIG. 1; and
FIG. 5D is a simplified outline view of the layers of FIGs. 5A-5C integrated into a single component (i.e., an engineered top tape) prior to assembly of an electrochemical-based analytical test strip according to the present invention;
FIG. 6 is a graph of fill speed (i.e., "timing" in milliseconds) versus enzyme extension for an electrochemical-based analytical test strip according to an embodiment of the present invention; and
FIG. 7 is a flow diagram depicting stages in a method for determining an analyte in a bodily fluid sample according to an embodiment of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein

In general, electrochemical-based analytical test strips for the determination of an analyte (such as glucose) in a bodily fluid sample (for example, whole blood) according to embodiments of the present invention include an electrically insulating substrate layer with a distal end and a patterned conductor layer that is disposed over the electrically-insulating substrate layer and has a working electrode and a counter/reference electrode. The electrochemical-based analytical test strips also include a patterned insulation layer with an electrode exposure window configured to expose a working electrode exposed portion and a counter/reference electrode exposed portion, a reagent layer, and a pattered spacer layer. In addition, the patterned insulation layer and the patterned spacer layer define a sample receiving chamber with a sample-receiving opening at the distal end of the electrically insulating substrate layer and that extends across the working electrode exposed portion and the counter/reference electrode exposed portion. Moreover, the reagent layer is disposed over the working electrode exposed portion and the counter/reference electrode exposed portion and extends no more than 400µm toward the sample-receiving opening beyond the distal most of the working electrode exposed portion and the counter/reference electrode exposed portion.

Electrochemical-based analytical test strips according to embodiments of the present invention are beneficial in that, for example, the fill speed of the electrochemical-based analytical test strip (e.g., the time for a bodily fluid sample to travel from one point to another point in a same-receiving chamber of the electrochemical-based analytical test strip (in this case it is the time taken for fluid to travel between a first working electrode and a second working electrode. The start and end times of the speed measurement are triggered by an increase in current beyond a pre-determined threshold - in this case the threshold current is 150nA) and fill speed variability are beneficially optimized. Reduction in fill speed reduces the risk of generating a fill speed-related error message during analyte determination (the error risk is related to the accuracy check performed by the meter on the end currents of the first and second working electrodes. If a strip fills too slowly, the end currents of the first and second working electrodes may be sufficiently different to cause an Error 5 message i.e. >20% difference in end current after 5 seconds) and also reduces the delay experienced by a user in receiving determination results. A reduction in fill speed variability reduces a user's perception of strip-to-strip variation that can cause concern or annoyance.

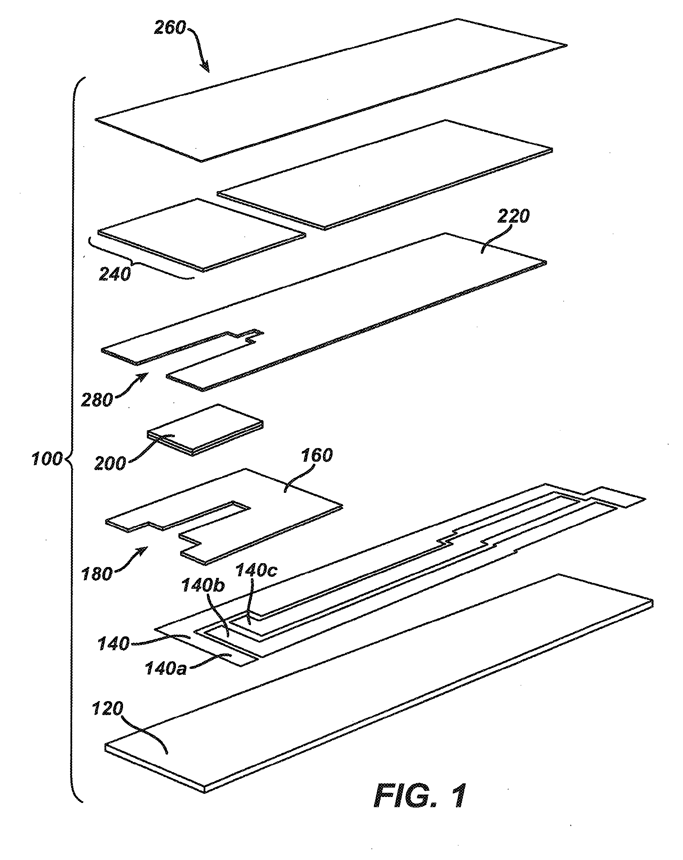

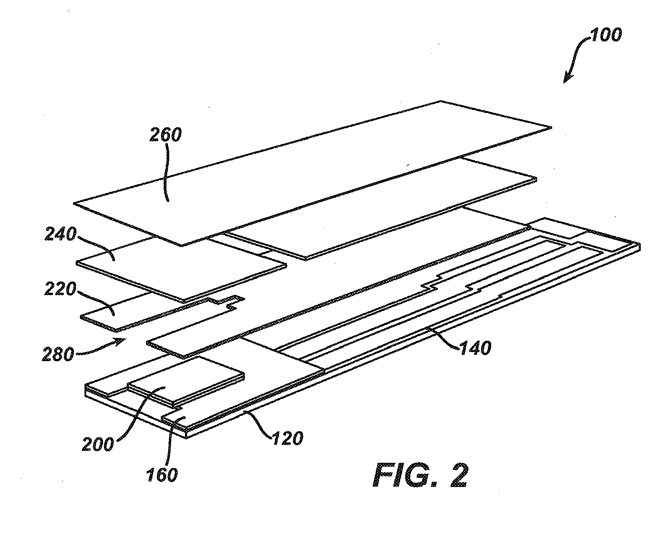

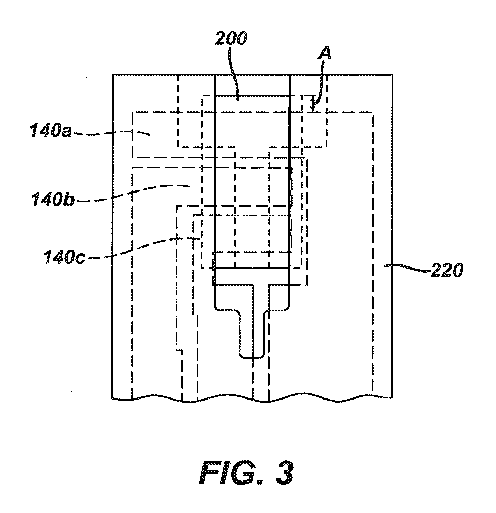

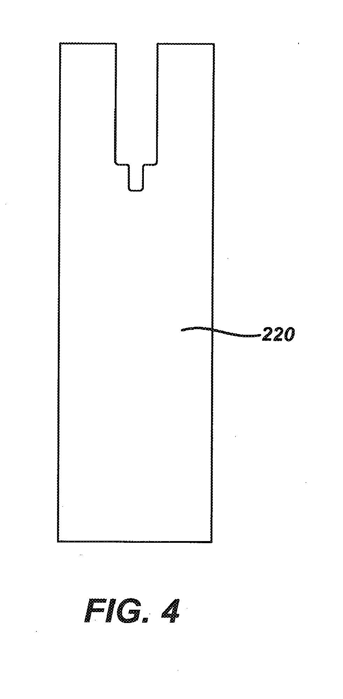

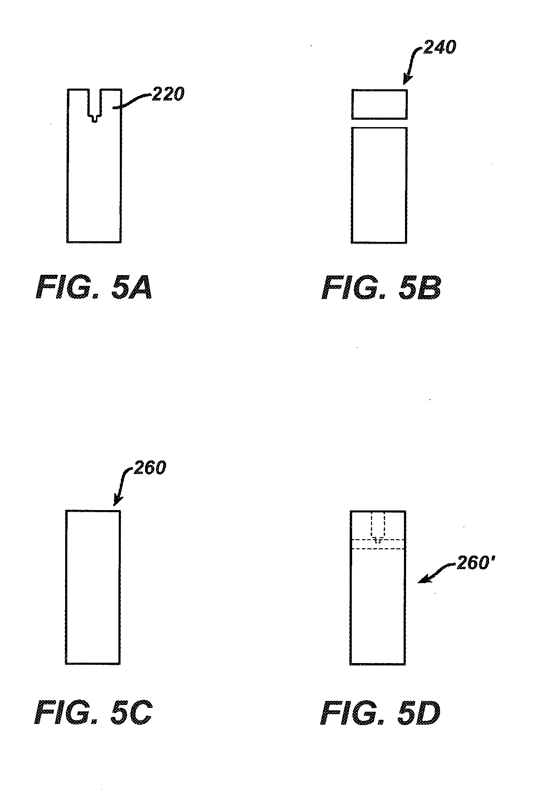

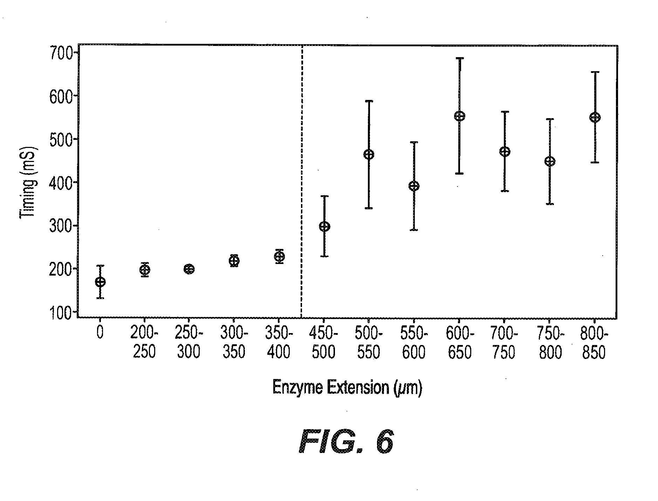

FIG. 1 is a simplified exploded view of an electrochemical-based analytical test strip according to an embodiment of the present invention. FIG. 2 is a simplified semi-exploded view of the electrochemical-based analytical test strip of FIG. 1. FIG. 3 is a simplified bottom outline view of a distal portion of an electrically insulating substrate layer, patterned conductor layer, patterned insulating layer, reagent layer, patterned spacer layer, and hydrophilic layer of the electrochemical-based analytical test strip of FIG. 1. FIG. 4 is a simplified top outline view of the patterned spacer layer, and hydrophilic layer of the electrochemical-based analytical test strip of FIG. 1. FIGs. 5A-5C are simplified top views of the patterned spacer layer, hydrophilic layer and top layer of the electrochemical-based analytical test strip of FIG. 1. FIG. 5D is a simplified outline view of the layers of FIGs. 5A-5C integrated into a single component (i.e., an engineered top tape) prior to assembly of an electrochemical-based analytical test strip according to the present invention. FIG. 6 is a graph of fill speed (i.e., "timing" in milliseconds) versus enzyme extension for an electrochemical-based analytical test strip according to an embodiment of the present invention.

Referring to FIGs. 1-6, electrochemical-based analytical test strip 100 for the determination of an analyte (such as glucose) in a bodily fluid sample (for example, a whole blood sample) includes an electrically-insulating substrate layer 120, a patterned conductor layer 140, a patterned insulation layer 160 with electrode exposure window 180 therein, an enzymatic reagent layer 200, a patterned spacer layer 220, a hydrophilic layer 240, and a top layer 260.

The disposition and alignment of electrically-insulating substrate layer 120, patterned conductor layer 140 (which includes a counter/reference electrode 140a, a first working electrode 140b and a second working electrode 140c, see FIGs. 1 and 3 in particular), patterned insulation layer 160, enzymatic reagent layer 200, patterned spacer layer 220, hydrophilic layer 240 and top layer 260 of electrochemical-based analytical test strip 100 are such that sample-receiving chamber 280 is formed within electrochemical-based analytical test strip 100.

Although, for the purpose of explanation only, electrochemical-based analytical test strip 100 is depicted as including three electrodes, embodiments of electrochemical-based analytical test strips, including embodiments of the present invention, can include any suitable number of electrodes.

Counter/reference electrode 140a, first working electrode 140b, and second working electrode 140c can be formed of any suitable material including, for example, gold, palladium, platinum, indium, titanium-palladium alloys and electrically conducting carbon-based materials. Referring in particular to FIG. 3, electrode exposure window 180 of patterned insulation layer 160 exposes a portion of counter/reference electrode 140a, a portion of first working electrode 140b and a portion of second working electrode 140c (such portions being specked in FIG. 3). During use, a bodily fluid sample is applied to electrochemical-based analytical test strip 100 and transferred to sample-receiving chamber 280, thereby operatively contacting the counter/reference electrode, first working electrode and second working electrode exposed portions.

Electrically-insulating substrate layer 120 can be any suitable electrically-insulating substrate layer known to one skilled in the art including, for example, a nylon substrate, polycarbonate substrate, a polyimide substrate, a polyvinyl chloride substrate, a polyethylene substrate, a polypropylene substrate, a glycolated polyester (PETG) substrate, or a polyester substrate. The electrically-insulating substrate layer can have any suitable dimensions including, for example, a width dimension of about 5 mm, a length dimension of about 27 mm and a thickness dimension of about 0.5 mm.

Electrically-insulating substrate layer 120 provides structure to the strip for ease of handling and also serves as a base for the application (e.g., printing or deposition) of subsequent layers (e.g., a patterned conductor layer). It should be noted that patterned conductor layers employed in analytical test strips according to embodiments of the present invention can take any suitable shape and be formed of any suitable materials including, for example, metal materials and conductive carbon materials.

Patterned insulation layer 160 can be formed, for example, from a screen printable insulating ink. Such a screen printable insulating ink is commercially available from Ercon of Wareham, Massachusetts U.S.A. under the name "Insulayer."

Patterned spacer layer 220 can be formed, for example, from a screen-printable pressure sensitive adhesive commercially available from Apollo Adhesives, Tamworth, Staffordshire, UK. In the embodiment of FIGs. 1 through 5C, patterned spacer layer 220 defines outer walls of the sample-receiving chamber 280.

Hydrophilic layer 240 can be, for example, a clear film with hydrophilic properties that promote wetting and filling of electrochemical-based analytical test strip 100 by a fluid sample (e.g., a whole blood sample). Such clear films are commercially available from, for example, 3M of Minneapolis, Minnesota U.S.A. If desired, patterned spacer layer 220, hydrophilic layer 240 and top layer 260 can be integrated into a single component 260' as depicted in FIG. 5D. Such an integrated component is also referred to as an Engineered Top Tape (ETT) and can be, for example, a pre-constructed laminate that defines the sides and top of the sample-receiving chamber. Suitable hydrophilic layers are commercially available from, for example, Coveme (San Lazzaro di Savena, Italy)

Enzymatic reagent layer 200 can include any suitable enzymatic reagents, with the selection of enzymatic reagents being dependent on the analyte to be determined. For example, if glucose is to be determined in a blood sample, enzymatic reagent layer 200 can include a glucose oxidase or glucose dehydrogenase along with other components necessary for functional operation. Enzymatic reagent layer 200 can include, for example, glucose oxidase, tri-sodium citrate, citric acid, polyvinyl alcohol, hydroxyl ethyl cellulose, potassium ferrocyanide, antifoam, cabosil, PVPVA, and water. Further details regarding enzymatic reagent layers, and electrochemical-based analytical test strips in general, are in U.S. Patent Nos. 6,241,862 and 6,733,655.

Referring to FIG. 3 in particular, enzymatic reagent layer 200 is disposed over the first and second working electrode exposed portions and the counter/reference electrode exposed portion and extends no more than 400µm toward the distal end of the sample-receiving opening beyond the distal most of the working electrode exposed portion and the counter/reference electrode exposed portion. In other words, the enzymatic reagent layer extends no more than 400µm upstream of the distal most electrode. This distance is demarcated by the arrow labeled "A" in FIG. 3. As described above and illustrated by the data of FIG. 6, limiting the extension of the enzymatic reagent layer to ≤ 400µm provides an unexpectedly slow fill speed and an unexpectedly low fill variability.

FIG. 6 is a graph of fill speed (i.e., "timing" in milliseconds) versus enzyme extension for an electrochemical-based analytical test strip according to an embodiment of the present invention. The data of FIG. 6 was collected using whole blood bodily fluid samples and an electrochemical-based analytical test strip with a sample-receiving chamber volume of 0.73 micro-liters, a sample-receiving chamber height of 0.130mm, a sample-receiving chamber length of 3.77mm and a primary sample-receiving chamber width of 1.50mm.

Referring to FIG. 6, it is evident that extensions of no more than 400µm and, in particularly, extensions in the range of 200µm to 400µm are unexpectedly beneficial with respect to optimizing (i.e., reducing) fill speed and fill speed variability.

It has been determined that electrochemical-based analytical test strips according to embodiments of the present invention are particularly beneficial with respect to optimizing fill speed and fill variability when the enzymatic reagent layer is relatively hydrophilic and/or has a chalky texture (i.e., has a powdery texture) prior to application of a bodily fluid sample to the electrochemical-based analytical test strip. It is hypothesized without being bound that chalky enzymatic reagent layers exhibit poor adhesion to the electrically-insulating substrate layer at a microscopic level that interferes with bodily fluid flow. Enzymatic reagent layers that contain silica can be relatively hydrophilic and/or have a chalky texture. Therefore, electrochemical-based analytical test strips according to embodiments of the present invention are also particularly beneficial when the enzymatic reagent layer contains silica.

Electrochemical-based analytical test strip 100 can be manufactured, for example, by the sequential aligned formation of patterned conductor layer 140, patterned insulation layer 160, enzymatic reagent layer 200, patterned spacer layer 220, hydrophilic layer 240 and top layer 260 onto electrically-insulating substrate layer 120. Any suitable techniques known to one skilled in the art can be used to accomplish such sequential aligned formation, including, for example, screen printing, photolithography, photogravure, chemical vapour deposition and tape lamination techniques.

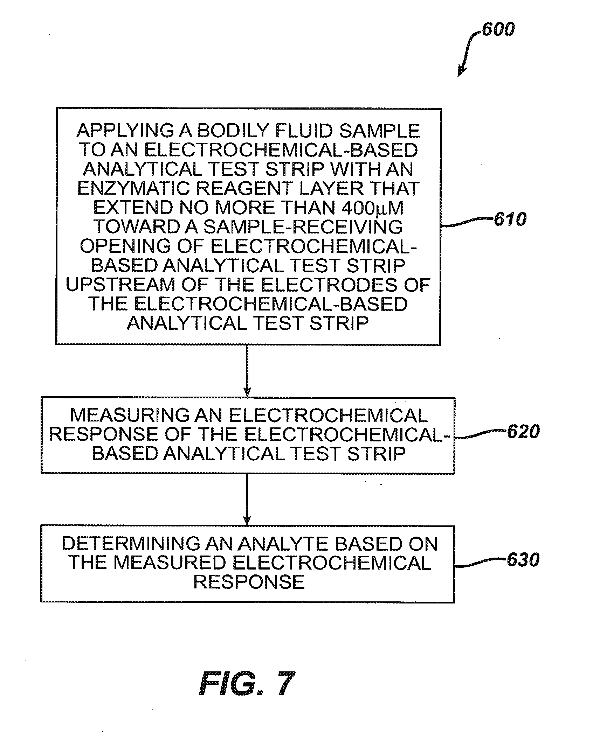

FIG. 7 is a flow diagram depicting stages in a method 600 for determining an analyte (such as glucose) in a bodily fluid sample according to an embodiment of the present invention. At step 610 of method 600, a bodily fluid sample is applied to an electrochemical-based analytical test strip such that the applied bodily fluid sample fills a sample-receiving chamber of the electrochemical-based analytical test strip. The electrochemical-based analytical test strip employed in step 610 has an electrically insulating substrate layer with a distal end and also has a patterned conductor layer (with a working electrode and a counter/reference electrode) that is disposed over the electrically-insulating layer. The electrochemical-based analytical test strip also has a patterned insulation layer with an electrode exposure window configured to expose a working electrode exposed portion and a counter/reference electrode exposed portion, an enzymatic reagent layer; and a patterned spacer layer. In addition, the patterned insulation layer and the patterned spacer layer define a sample receiving chamber with a sample-receiving opening at the distal end of the electrically insulating base layer and that extends across the working electrode exposed portion and the counter/reference electrode exposed portion. Moreover, the enzymatic reagent layer is disposed over the working electrode exposed portion and the counter/reference electrode exposed portion and extends no more than 400µm toward the sample-receiving opening beyond the distal most of the working electrode exposed portion and the counter/reference electrode exposed portion. In other words, the enzymatic reagent layer extends no more than 400µm upstream of the electrodes of the electrochemical-based analytical test strip as previously discussed with respect to FIG. 3.

Method 600 also includes measuring an electrochemical response of the electrochemical-based analytical test strip (see step 620 Of FIG. 7) and, at step 630, determining the analyte based on the measured electrochemical response. The measuring and determination steps (i.e., steps 620 and 630) can, if desired, by performed using a suitable associated meter.

Once apprised of the present disclosure, one skilled in the art will recognize that method 600 can be readily modified to incorporate any of the techniques, benefits and characteristics of electrochemical-based analytical test strips according to embodiments of the present invention and described herein.

## Claims

1. An electrochemical-based analytical test strip for the determination of an analyte in a bodily fluid sample, the electrochemical-based analytical test strip comprising:
an electrically insulating substrate layer with a distal end;
a patterned conductor layer disposed over the electrically-insulating substrate layer, the patterned conductive layer including at least a working electrode and a counter/reference electrode;
a patterned insulation layer with an electrode exposure window configured to expose a working electrode exposed portion and a counter/reference electrode exposed portion
an enzymatic reagent layer; and
a patterned spacer layer,
wherein the patterned insulation layer and the patterned spacer layer define a sample receiving chamber with a sample-receiving opening at the distal end of the electrically insulating substrate layer and that extends across the working electrode exposed portion and the counter/reference electrode exposed portion, and
wherein the enzymatic reagent layer is disposed over the working electrode exposed portion and the counter/reference electrode exposed portion and extends no more than 400µm toward the sample-receiving opening beyond the distal most of the working electrode exposed portion and the counter/reference electrode exposed portion.

2. The electrochemical-based analytical test strip of claim 1 wherein the analyte is glucose and the bodily fluid sample is blood.

3. A method for determining an analyte in a bodily fluid sample, the method comprising:
applying a bodily fluid sample to an electrochemical-based analytical test strip such that the applied bodily fluid sample fills a sample-receiving chamber of the electrochemical-based analytical test strip, the electrochemical-based analytical test strip having:
an electrically insulating substrate layer with a distal end;
a patterned conductor layer disposed over the electrically-insulating substrate layer, the patterned conductive layer including at least a working electrode and a counter/reference electrode;
a patterned insulation layer with an electrode exposure window configured to expose a working electrode exposed portion and a counter/reference electrode exposed portion;
an enzymatic reagent layer; and
a patterned spacer layer,
wherein the patterned insulation layer and the patterned spacer layer define the sample receiving chamber with a sample-receiving opening at the distal end of the electrically insulating substrate layer and that extends across the working electrode exposed portion and the counter/reference electrode exposed portion, and
wherein the reagent layer is disposed over the working electrode exposed portion and the counter/reference electrode exposed portion and extends no more than 400µm toward the sample-receiving opening beyond the distal most of the working electrode exposed portion and the counter/reference electrode exposed portion;
measuring an electrochemical response of the electrochemical-based analytical test strip; and
determining the analyte based on the measured electrochemical response.

4. The method of claim 3 wherein the bodily fluid sample is whole blood.

5. The method of claim 3 wherein the analyte is glucose.

6. The test strip of claim 1 or the method of claim 3 wherein the patterned spacer layer is formed of a hydrophilic material.

7. The test strip of claim 1 or the method of claim 3 wherein the enzymatic reagent layer extends a distance in the range of 200µm to 400µm.

8. The test strip of claim 1 or the method of claim 3 wherein the patterned conductor layer includes a first working electrode, a second working electrode and a counter/reference electrode.

9. The test strip of claim 1 or the method of claim 3 wherein the enzymatic reagent layer has a chalky texture.

10. The test strip of claim 1 or claim 9, or the method of claim 3 or claim 9 wherein the enzymatic reagent layer contains silica.

11. The test strip of claim 1 or the method of claim 3 wherein the electrochemical-based analytical test strip further includes:
a hydrophilic layer; and
a top layer.

12. The test strip of claim 1 or the method of claim 11 wherein the patterned spacer layer, hydrophilic layer and top layer are integrated into a single component.

## Patentansprüche

1. Analytischer Teststreifen auf elektrochemischer Basis zum Bestimmen eines Analyten in einer Körperflüssigkeitsprobe, wobei der analytische Teststreifen auf elektrochemischer Basis umfasst:
eine elektrisch isolierende Substratschicht mit einem distalen Ende;
eine strukturierte Leiterschicht, die über der elektrisch isolierenden Substratschicht angeordnet ist, wobei die strukturierte Leiterschicht wenigstens eine Arbeitselektrode und eine Gegen/Referenzelektrode umfasst;
eine strukturierte Isolatorschicht mit einem Elektrodenexpositionsfenster, das dafür gestaltet ist, einen exponierten Teil der Arbeitselektrode und einen exponierten Teil der Gegen/Referenzelektrode zu exponieren;
eine Enzymreagensschicht; und
eine strukturierte Abstandshalterschicht,
wobei die strukturierte Isolatorschicht und die strukturierte Abstandshalterschicht eine Probenaufnahmekammer definieren, die eine Probenaufnahmeöffnung an dem distalen Ende der elektrisch isolierenden Substratschicht aufweist und die sich über den exponierten Teil der Arbeitselektrode und den exponierten Teil der Gegen/Referenzelektrode erstreckt, und
wobei die Enzymreagensschicht über dem exponierten Teil der Arbeitselektrode und dem exponierten Teil der Gegen/Referenzelektrode angeordnet ist und sich um nicht mehr als 400 µm über das am weitesten distale von dem exponierten Teil der Arbeitselektrode und dem exponierten Teil der Gegen/Referenzelektrode hinaus zu der Probenaufnahmeöffnung erstreckt.

2. Analytischer Teststreifen auf elektrochemischer Basis gemäß Anspruch 1, wobei der Analyt Glucose ist und die Körperflüssigkeitsprobe Blut ist.

3. Verfahren zum Bestimmen eines Analyten in einer Körperflüssigkeitsprobe, wobei das Verfahren umfasst:
Aufbringen einer Körperflüssigkeitsprobe auf einen analytischen Teststreifen auf elektrochemischer Basis, so dass die aufgebrachte Körperflüssigkeitsprobe eine Probenaufnahmekammer des analytischen Teststreifens auf elektrochemischer Basis füllt, wobei der analytische Teststreifen auf elektrochemischer Basis aufweist:
eine elektrisch isolierende Substratschicht mit einem distalen Ende;
eine strukturierte Leiterschicht, die über der elektrisch isolierenden Substratschicht angeordnet ist, wobei die strukturierte Leiterschicht wenigstens eine Arbeitselektrode und eine Gegen/Referenzelektrode umfasst;
eine strukturierte Isolatorschicht mit einem Elektrodenexpositionsfenster, das dafür gestaltet ist, einen exponierten Teil der Arbeitselektrode und einen exponierten Teil der Gegen/Referenzelektrode zu exponieren;
eine Enzymreagensschicht; und
eine strukturierte Abstandshalterschicht,
wobei die strukturierte Isolatorschicht und die strukturierte Abstandshalterschicht eine Probenaufnahmekammer definieren, die eine Probenaufnahmeöffnung an dem distalen Ende der elektrisch isolierenden Substratschicht aufweist und die sich über den exponierten Teil der Arbeitselektrode und den exponierten Teil der Gegen/Referenzelektrode erstreckt, und
wobei die Reagensschicht über dem exponierten Teil der Arbeitselektrode und dem exponierten Teil der Gegen/Referenzelektrode angeordnet ist und sich um nicht mehr als 400 µm über das am weitesten distale von dem exponierten Teil der Arbeitselektrode und dem exponierten Teil der Gegen/Referenzelektrode hinaus zu der Probenaufnahmeöffnung erstreckt;
Messen einer elektrochemischen Antwort des analytischen Teststreifens auf elektrochemischer Basis; und
Bestimmen des Analyten auf der Basis der gemessenen elektrochemischen Antwort.

4. Verfahren gemäß Anspruch 3, wobei die Körperflüssigkeitsprobe Vollblut ist.

5. Verfahren gemäß Anspruch 3, wobei der Analyt Glucose ist.

6. Teststreifen gemäß Anspruch 1 oder Verfahren gemäß Anspruch 3, wobei die strukturierte Abstandshalterschicht aus einem hydrophilen Material besteht.

7. Teststreifen gemäß Anspruch 1 oder Verfahren gemäß Anspruch 3, wobei sich die Enzymreagensschicht über einen Abstand in dem Bereich von 200 µm bis 400 µm erstreckt.

8. Teststreifen gemäß Anspruch 1 oder Verfahren gemäß Anspruch 3, wobei die strukturierte Leiterschicht eine erste Arbeitselektrode, eine zweite Arbeitselektrode und eine Gegen/Referenzelektrode umfasst.

9. Teststreifen gemäß Anspruch 1 oder Verfahren gemäß Anspruch 3, wobei die Enzymreagensschicht eine kreideartige Textur aufweist.

10. Teststreifen gemäß Anspruch 1 oder Anspruch 9 oder Verfahren gemäß Anspruch 3 oder Anspruch 9, wobei die Enzymreagensschicht Siliciumoxid enthält.

11. Teststreifen gemäß Anspruch 1 oder Verfahren gemäß Anspruch 3, wobei der analytische Teststreifen auf elektrochemischer Basis ferner umfasst:
eine hydrophile Schicht; und
eine Deckschicht.

12. Teststreifen gemäß Anspruch 1 oder Verfahren gemäß Anspruch 11, wobei die strukturierte Abstandshalterschicht, die hydrophobe Schicht und die Deckschicht zu einer einzelnen Komponente integriert sind.

## Revendications

1. Bandelette de test analytique à base électrochimique pour la détermination d'un analyte dans un échantillon de fluide corporel, la bandelette de test analytique à base électrochimique comprenant :
une couche de substrat électriquement isolante avec une extrémité distale ;
une couche conductrice structurée disposée sur la couche de substrat électriquement isolante, la couche conductrice structurée comportant au moins une électrode de travail et une contre-électrode/électrode de référence ;
une couche d'isolation structurée avec une fenêtre d'exposition d'électrode configurée pour exposer une partie exposée d'électrode de travail et une partie exposée de contre-électrode/électrode de référence ;
une couche de réactifs enzymatiques ; et
une couche d'entretoise structurée,
dans laquelle la couche d'isolation structurée et la couche d'entretoise structurée définissent une chambre de réception d'échantillon avec une ouverture de réception d'échantillon à l'extrémité distale de la couche de substrat électriquement isolante et qui s'étend à travers la partie exposée d'électrode de travail et la partie exposée de contre-électrode/électrode de référence, et
dans laquelle la couche de réactifs enzymatiques est disposée sur la partie exposée d'électrode de travail et la partie exposée de contre-électrode/électrode de référence et ne s'étend pas sur plus de 400 µm vers l'ouverture de réception d'échantillon au-delà de la plus distale de la partie exposée d'électrode de travail et de la partie exposée de contre-électrode/électrode de référence.

2. Bandelette de test analytique à base électrochimique de la revendication 1 dans laquelle l'analyte est le glucose et l'échantillon de fluide corporel est le sang.

3. Procédé de détermination d'un analyte dans un échantillon de fluide corporel, le procédé comprenant :
l'application d'un échantillon de fluide corporel à une bandelette de test analytique à base électrochimique de telle sorte que l'échantillon de fluide corporel appliqué remplisse une chambre de réception d'échantillon de la bandelette de test analytique à base électrochimique, la bandelette de test analytique à base électrochimique ayant :
une couche de substrat électriquement isolante avec une extrémité distale ;
une couche conductrice structurée disposée sur la couche de substrat électriquement isolante, la couche conductrice structurée comportant au moins une électrode de travail et une contre-électrode/électrode de référence ;
une couche d'isolation structurée avec une fenêtre d'exposition d'électrode configurée pour exposer une partie exposée d'électrode de travail et une partie exposée de contre-électrode/électrode de référence ;
une couche de réactifs enzymatiques ; et
une couche d'entretoise structurée,
la couche d'isolation structurée et la couche d'entretoise structurée définissant la chambre de réception d'échantillon avec une ouverture de réception d'échantillon à l'extrémité distale de la couche de substrat électriquement isolante et qui s'étend à travers la partie exposée d'électrode de travail et la partie exposée de contre-électrode/électrode de référence, et
la couche de réactifs étant disposée sur la partie exposée d'électrode de travail et la partie exposée de contre-électrode/électrode de référence et ne s'étendant pas sur plus de 400 µm vers l'ouverture de réception d'échantillon au-delà de la plus distale de la partie exposée d'électrode de travail et de la partie exposée de contre-électrode/électrode de référence ;
la mesure d'une réponse électrochimique de la bandelette de test analytique à base électrochimique ; et
la détermination de l'analyte d'après la réponse électrochimique mesurée.

4. Procédé de la revendication 3 dans lequel l'échantillon de fluide corporel est du sang total.

5. Procédé de la revendication 3 dans lequel l'analyte est le glucose.

6. Bandelette de test de la revendication 1 ou procédé de la revendication 3, la couche d'entretoise structurée étant constituée d'un matériau hydrophile.

7. Bandelette de test de la revendication 1 ou procédé de la revendication 3, la couche de réactifs enzymatiques s'étendant sur une distance dans la gamme de 200 µm à 400 µm.

8. Bandelette de test de la revendication 1 ou procédé de la revendication 3, la couche conductrice structurée comportant une première électrode de travail, une deuxième électrode de travail et une contre-électrode/électrode de référence.

9. Bandelette de test de la revendication 1 ou procédé de la revendication 3, la couche de réactifs enzymatiques ayant une texture crayeuse.

10. Bandelette de test de la revendication 1 ou la revendication 9, ou procédé de la revendication 3 ou la revendication 9, la couche de réactifs enzymatiques contenant de la silice.

11. Bandelette de test de la revendication 1 ou procédé de la revendication 3, la bandelette de test analytique à base électrochimique comportant en outre :
une couche hydrophile ; et
une couche supérieure.

12. Bandelette de test de la revendication 1 ou procédé de la revendication 11, la couche d'entretoise structurée, la couche hydrophile et la couche supérieure étant intégrées en un seul composant.
